# EUROPEAN PATENT APPLICATION

(11) **EP 2 311 961 A1**
(43) Date of publication of application: **20.04.2011**
(21) Application number: 09173197.6
(22) Date of filing: 15.10.2009
(51) Int. Cl.: C12N 15/63, C12N 15/64

(54) **Model-guided random assembly PCR for the synthesis of eukaryotic promoters**

(71) Applicant: DKFZ Deutsches Krebsforschungszentrum, 69120 Heidelberg (DE); Ruprecht-Karls-Universität Heidelberg, 69117 Heidelberg (DE)
(72) Inventor: Reis, Yara, 69121 Heidelberg (DE); Richter, Daniela, 44267 Dortmund (DE); Reichenzeller, Michaela, 88447 Warthausen (DE); Iwamoto, Nao, 69121 Heidelberg (DE); Zhu, Chenchen, 69120 Heidelberg (DE); Uckelmann, Hannah, 69120 Heidelberg (DE); Eils, Roland, 69198 Schriesheim (DE); Keienburg, Jens, 69221 Dossenheim (DE); Hundeshagen, Philipp, 69126 Heidelberg (DE); Bernardo, Marti, 69115 Heidelberg (DE); Bauer, Tobias, 69123 Heidelberg (DE); Kranz, Anna-Lena, 69120 Heidelberg (DE); König, Rainer, 69221 Dossenheim (DE); Heinemann, Tim, 69221 Dossenheim (DE); Mugahid, Douaa, 69115 Heidelberg (DE); Velten, Lars, 69121 Heidelberg (DE); Haas, Simon, 69123 Heidelberg (DE); Hiller, Corinna, 74243 Langenbrettach (DE); Bartoschek, Michael, 69120 Heidelberg (DE); Rademacher, Anne, 69124 Heidelberg (DE); Meyer, Hannah, 69121 Heidelberg (DE); Krämer, Stephen, 69221 Dossenheim (DE); Zhao, Bingging, 69120 Heidelberg (DE)
(74) Representative: Dick, Alexander

(57) **Abstract**

The current invention is concerned with a method for the manufacture of a polynucleotide comprising a synthetic promoter specifically controlled by at least one selected transcription factor, comprising a) determining the distance between the transcriptional start point and the binding site for said at least one transcription factor in a given number of promoters comprising at least one binding site for said at least one transcription factor, b) selecting a distance range comprising the distances determined in a) to occur most frequently, c) determining the accompanying transcription factors in a given number of promoters, d) selecting a suitable number of accompanying transcription factors determined in c) to occur most frequently, e) synthesizing the synthetic promoter by randomly assembling i) at least one binding site for each of the at least one transcription factor, ii) at least one binding site for each of the selected accompanying transcription factor(s), and iii) a suitable number of stuffer fragments and by arranging said randomly assembled binding sites in the distance range from the transcriptional start point selected in b). The invention is further concerned with a polynucleotide comprising the synthetic promoter manufactured by the method of the current invention, a vector comprising the polynucleotide of the current invention, and a cell comprising the polynucleotide or the vector of the current invention. Furthermore, the invention relates to a device for the manufacture of oligonucleotides suitable for manufacturing a synthetic promoter of the current invention and a device for the manufacture of the synthetic promoter of the current invention.

## Description

The current invention is concerned with a method for the manufacture of a polynucleotide comprising a synthetic promoter specifically controlled by at least one selected transcription factor, comprising a) determining the distance between the transcriptional start point and the binding site for said at least one transcription factor in a given number of promoters comprising at least one binding site for said at least one transcription factor, b) selecting a distance range comprising the distances determined in a) to occur most frequently, c) determining the accompanying transcription factors in a given number of promoters, d) selecting a suitable number of accompanying transcription factors determined in c) to occur most frequently, e) synthesizing the synthetic promoter by randomly assembling i) at least one binding site for each of the at least one transcription factor, ii) at least one binding site for each of the selected accompanying transcription factor(s), and iii) a suitable number of stuffer fragments and by arranging said randomly assembled binding sites in the distance range from the transcriptional start point selected in b). The invention is further concerned with a polynucleotide comprising the synthetic promoter manufactured by the method of the current invention, a vector comprising the polynucleotide of the current invention, and a cell comprising the polynucleotide or the vector of the current invention. Furthermore, the invention relates to a device for the manufacture of oligonucleotides suitable for manufacturing a synthetic promoter of the current invention and a device for the manufacture of the synthetic promoter of the current invention.

Promoters are the key regulators of gene expression. Possessing promoters which are active under a desired condition and / or in a specified tissue is of great value for biotechnology and molecular biology, including plant biotechnology, gene therapy and fundamental research in bioscience. Most efforts of obtaining such promoters focus on obtaining such promoters from nature (examples are shown in [1] and[2]).

However, this approach has severeal disadvantages: First, promoters in eukaryotic cells are very complexly regulated by a wide variety of transcription factors and pathways.Therefore, natural promoters cannot be used reliably in transcriptional assays. Second, it may be desirable to have in hand a promoter which is active under a set of conditions for which no natural promoter exists. Third, for a precise control of gene expression levels, promoters of defined expression strengths are required.

Therefore, it has become a desire to synthetically construct promoters. Many efforts have been made to model promoters in silico in order to get information about their composition (reviewed in [3]). In short, such models determine the distribution of transcription factor binding sites (TFBS) along promoters by performing a bioinformatic analysis over orthologous sequence data. However, as yet it has not has been verified that promoters derived from such models do work in vivo.

Efforts to synthesize promoters biochemically [4], [5] do not have the potential to be scaled up towards synthesizing promoters of complex regulation. These methods rely on cloning repeats of a certain cis-regulatory motif or a randomized combination of cis-regulatory elements directly 5' of the core promoter.

Thus, there is a need in the art to obtain promoters which are responsive to defined stimuli by an increase or decrease in transcriptional activity.

The present invention, now, relates to a a method for the manufacture of a polynucleotide comprising a synthetic promoter specifically controlled by at least one selected transcription factor, comprising a) determining the distance between the transcriptional start point and the binding site for said at least one transcription factor in a given number of promoters comprising at least one binding site for said at least one transcription factor, b) selecting a distance range comprising the distances determined in a) to occur most frequently, c) determining the accompanying transcription factors in a given number of promoters, d) selecting a suitable number of accompanying transcription factors determined in c) to occur most frequently, e) synthesizing the synthetic promoter by randomly assembling i) at least one binding site for each of the at least one transcription factors, ii) at least one binding site for each of the selected accompanying transcription factor(s),, and iii) a suitable number of stuffer fragments and by arranging said randomly assembled binding sites in the distance range from the transcriptional start point selected in b).
The term "polynucleotide" as used in the current specification, preferably, relates to DNA, including cDNA. The term encompasses single as well as double stranded polynucleotides. Moreover, comprised are also chemically modified polynucleotides including naturally occurring modified polynucleotides such as glycosylated or methylated polynucleotides or artificial modified ones such as biotinylated polynucleotides. The polynucleotide of the present invention shall be provided, preferably, either as an isolated polynucleotide (i.e. isolated from its natural context) or in genetically modified form.

The term "transcription factor" as used herein relates to a cellular factor regulating gene transcription. Preferably, said factor is a polypeptide with the ability to bind to a specific nucleic acid sequence, i.e. the binding site, which is specific for a specific transcription factor. It is to be understood, however, that the binding site need not always be defined by a unique nucleotide sequence. Thus, nucleotide sequences comprising ambiguously defined nucleotides, e.g. due to a lack of exact knowledge in the art or due to unspecific binding of the transcription factor, are also included as binding sites.

As used herein, the term "synthetic promoter" relates to a polynucleotide comprising at least one binding site of a transcription factor and, preferably, excluding binding sites for other transcription factors. Preferably, the synthetic promoter comprises at least two different binding sites for at least two different transcription factors. More preferably, the synthetic promoter comprises a multitude, e.g. two, three, four, five, six, seven, eight, nine, or ten instances for each binding site it comprises. It is to be understood that the synthetic promoter may comprise other nucleic acid sequences as well, e.g. stuffer fragments that do not mediate binding of a transcrition factor but allow for a correct spatial arrangement of binding sites. The synthetic promoter is synthesized using the method of the current specification, thus is synthesized artificially; it is to be understood, however, that the sequence of the synthetic promoter may, by cause of the statistical elements involved in the synthesis method specified herein, be identical with a naturally occurring promoter.

The term "synthetic promoter specifically controlled by at least one selected transcription factor ", preferably, relates to a synthetic promoter mediating an increase, or decrease, as the case may be, in transcription from said promoter mediated by only the transcription factor or the transcription factors for which binding sites have been incorporated into said promoter. It is to be understood, however, that influence from other transcription factors, e.g. general transcription factors or transcription factors binding promiscously to polynucleotides, is not always excluded. Thus, a synthetic promoter wherein at least 50%, at least 60%, at least 70%, at least 80%, at least 90%, at least 95%, or at least 99% of transcription activitiy or repression activity is mediated by the transcription factor or the transcription factors for which binding sites have been incorporated into said promoter is to be considered to be specifically controlled by said transcription factor or transcription factors.

A "selected transcription factor" as used herein is a transcription factor willingly selected as the transcription factor to be used in the regulation of the synthetic promoter of the currrent invention. Criteria for the selection may, e.g., be scientific interest, intended tissue-specific or environment-specific control of transcription, or the like.

The term "distance between the transcriptional start point and the binding site" (distance) as used herein, preferably, relates to the number of nucleotides between the first nucleotide transcribed by the RNA polymerase (transcriptional start point) and the first nucleotide of the binding site. It is to be understood that there may be more than one transcriptional start points and also that there may be more than one binding site in a given promoter, thus there may be more than one distance to be determined in a given promoter.

As used in this specification, the term "given number of promoters", preferably, relates to a number of promoters suitable for obtaining statistical data on the distances between the transcriptional start points and the binding sites occurring in natural promoters. Preferably, said number is more than five, more than ten, more than twenty, more than thirty, or more than fifty naturally occurring promoters comprising at least one binding site for a selected transcription factor. More preferably, said distance is determined for all occurrances of binding sites in a complete genome.

The term "distance range comprising the distances to occur most frequently" relates to a range of distances comprising the distances that were obtained most frequently in a given number of promoters. Preferably, said distance range is selected by calculating the mean distance obtainable from a given number of promoters and by selecting the mean distance +/- 50% of the mean distance, +/- 40% of the mean distance, +/- 30% of the mean distance, +/- 20% of the mean distance, +/- 10% of the mean distance, or +/- 5% of the mean distance, rounded to the next natural number. It is, however, also contemplated by the current specification that the distribution of distances is more than one-phasic, i.e. has more than one maximum, of which one or more may be lower than others, i.e. the distribution may comprise main and side maxima. In such case the distance range may be selected as specified above from at least one of the main maxima and / or at least one of the side maxima.

The term "accompanying transcription factors" as used herein relates to transcription factors whose binding sites are correlated to binding sites of selected transcription factors in naturally occurring promoters in a statistically significant way. The determination of accompanying transcription factors comprises counting occurrances of binding sites for transcription factors with known binding sites in naturally occurring promoters comprising binding sites of selected transcription factors (coincidence analysis). Preferably, the ten, nine, eight, seven, six, five, four, three, or two transcription factors for which the highest number of occurrances in such determination is obtained, are accompanying transcription factors. It is to be contemplated that the transcription factor with the highest number of correlating binding sites typically will be the selected transcription factor, thus the selected transcription factor is to be excluded from the accompanying transcription factors.

Selecting a suitable number of accompanying transcription factors determined to occur most frequently comprises choosing one or more transcription factors from the list of accompanying transcription factors obtained as specified above. It is, however, also contemplated by the current invention that the number of accompanying transcription factors can be zero. Said selection may be knowledge-based or statistical, preferably said selection is based on the frequency of binding sites counted. More preferably, the at least one accompanying transcription factor selected is / are the transcription factor(s) with the highest frequency of binding sites in naturally occurring promoters comprising binding sites of selected transcription factors.

As used herein, the term "synthesizing the synthetic promoter" comprises synthesizing a polynucleotide comprising the nucleic acid sequences as specified herein. Synthesis may e.g. be accomplished by chemical or by biochemical means, e.g. by using a template-directed polynucleic acid synthesizing enzyme, preferably a DNA polymerase, more preferably a heat-stable DNA-polymerase, most preferably a Taq or a Pfu-DNA polymerase.

Preferably, the synthetic promoter comprises at least one binding site for each of the at least one selected transcription factors, thus, said promoter may also comprise more than one binding site for each of the selected transcription factors. The synthetic promoter further comprises at least one binding site for each of the selected accompanying transcription factor or the selected accompanying transcription factors. Furthermore, said promoter comprises a suitable number of stuffer fragments; said stuffer fragments may comprise the same sequence in all occurrences of a stuffer fragment or stuffer fragments may differ in sequence. A suitable number of stuffer fragments is at least one, but no more than 60% of total fragments.

The term "randomly assembling" as used herein, preferably relates to an assembly of the nucleic acid sequences as specified herein above in a way that allows statistical distribution of the nucleic acid sequences over the sequence of the synthetic promoter. Preferably, said statistical distribution is accomplished by synthesizing said polynucleotide comprising a synthetic promoter by random assembly PCR, using oligonucleotides comprising the nucleotide sequence of a binding site for a selected transcription factor and the nucleotide sequence of a binding site for an accompanying transcription factor or the nucleotide sequence of a stuffer fragment as primers (see example 1) The method of random assembly PCR developed in this specification is a further development of a method described in [8]. A core promoter comprising at least a TATA-boxs is required. Any core promoter, be it natural or synthetic, is sufficient. A suitable core promoter, e.g., is the core promoter of the Cytomegalovirus promoter, which can be cloned from Cytomegalovirus DNA by using the Oligonucleotides GGCGGTAGGCGTGTA (forward) and atttcgataagccagtaagc (reverse) as PCR primers.

As used herein, the term "arranging binding sites" relates to a spatial arrangement of the synthetic promoter manufactured according to the method as specified herein relative to the transcriptional start point. Preferably, said arrangement comprises cloning of a polynucleotide between the synthetic promoter and the transcription start point. Said polynucleotide is selected to have a length that arranges the synthetic promoter such that half of its nucleotides are closer, and half of its nucleotides are more distant than the mean distance obtained in the selection of a distance range as specified herein above. More preferably, said arrangement allows for some variation in the distance of the synthetic promoter from the transcriptional start point, i.e. half of the nucleotides are closer, and half of the nucleotides are more distant than the mean distance +/- 50% of the mean distance, +/- 40% of the mean distance, +/- 30% of the mean distance, +/- 20% of the mean distance, +/- 10% of the mean distance, or +/- 5% of the mean distance. Preferably, said polynucleotide between the synthetic promoter and the transcription start point does not comprise a binding site for a transcription factor known in the art.

In a preferred embodiment, the current invention relates to a polynucleotide comprising the synthetic promoter manufactured by the methods specified herein above.

In another preferred embodiment, the current invention relates to a vector comprising the polynucleotide the current specification.

The term "vector", preferably, encompasses phage, plasmid, viral or retroviral vectors as well artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotides of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques or by using naturally occurring transformation mechanisms like e.g. the tranfer of the Ti-plasmid or its derivatives by Agrobacterium species. Should the vector be a virus, it may be packaged in vitro using an appropriate packaging cell line prior to application to host cells. Retroviral vectors may be replication competent or replication defective. In the latter case, viral propagation generally will occur only in complementing hosts/cells.

Preferably, the vector of the invention comprises additional expression control sequences allowing expression in prokaryotic or eukaryotic cells or isolated fractions thereof. Regulatory elements ensuring expression in eukaryotic cells are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Moreover, inducible expression control sequences may be used in conjunction with the synthetic promoter of the present invention in an expression vector encompassed by the present invention. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (InVitrogene) or SPORT1 (GIBCO BRL). Preferably, said vector is a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into the targeted cell population. Methods which are well known to those skilled in the art can be used to construct recombinant viral vectors; see, for example, the techniques described in [6].

A further preferred embodiment of the current invention is a host cell comprising the polynucleotide or the vector of the current invention.

As used herein, a "host cell" is a cell comprising at least one of the selected transcription factors. Preferably, the cell is an eukaryotic cell. More preferably, the cell is a mammalian cell. It is, however, also contemplated by the current invention that the cell is a plant cell, preferably a tracheophyte cell, more preferably a spermatophyte cell. The host cell is either a cultured cell or a body cell. More preferably, the host cell is comprised in an organism, e.g. an archaebacterium, a bacterium, a plant, an animal including humans.

In another preferred embodiment, the current invention relates to a device for the manufacture of oligonucleotides suitable for manufacturing a synthetic promoter, comprising a) a database comprising data from a coincidence analysis of transcription factors and b) a synthesis unit synthesizing oligonucleotides, wherein the nucleotide sequence of said oligonucleotides is determined using data comprised in the database of a).

A "database" in accordance with the present invention refers to a collection of information (i.e. data) which is stored on a suitable medium in a systematic way. Said collection of information may be stored on a single storage medium or on physically separated separate storage media operatively linked to each other. If the information is stored on physically separate storage media, e.g., due to fragmentation of the information, it is envisaged that the partial information stored on each of said media can be reassembled so that the collection of information in its entirety can be investigated, e.g., by a query search. Suitable storage media for information include entire computers, data processing devices or isolated magnetic, optical or other storage media such as hard disks, CDs, CD-ROMs, MP-3 and the like. The database may further comprise a database management system. Database management systems may be on the basis of a network model, a hierarchical model, a relational model or an object-oriented database model. An alternative database management system may be based on the so-called fuzzy logic. It is envisaged that the database referred to in accordance with the present invention has a structure which allows data analysis by a suitable analyzing tool, e.g., a computer implemented search algorithm, in order to answer queries regarding the collection of information stored in the said database.

The term "device" as used herein relates to a system of means comprising at least the means operatively linked to each other as to allow the manufacture. Preferred means for synthesizing oligonucleotides are well known in the art. How to link the means in an operating manner will depend on the type of means included into the device. Preferably, the means are comprised by a single device in such a case. Said device may accordingly include a synthesis unit for the synthesis of oligonucleotides and a computer unit for processing data from the database for the selection of binding site sequences and of accompanying transcription factors and their binding sites.

Preferably, the device further comprises means for synthesizing a polynucleotide comprising a synthetic promoter. The synthesis unit is, e.g. a PCR unit randomly combining nucleic acid sequences obtained by selection from the databases in addition to a suitable number of stuffer fragments as specified herein above.

More preferably, the device comprises both the means for synthesizing oligonucleotides and the means for synthesizing a polynucleotide comprising a synthetic promoter operatively linked in one device. Thus, the oligonucleotides synthesized means for synthesizing oligonucleotides are transferred to said means for synthesizing a polynucleotide, preferably without further treatment or purification.

However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician,

### References

[1] Schmidt, M. et al in Eur Arch Otorhinolaryngology 261 (2004), p. 208-15: "Inducible promoters for gene therapy of head and neck cancer: an in vitro study"
[2] Atkinson, H.J. in Annual Review of Phytopathology 41 (2003), p. 615-639: "Engineering plants for nematode resistance"
[3] Venter, M. in Trends in Plant Science 12 (2007), p. 118-124: "Synthetic promoters: genetic control through cis engineering" (an the references cited therein)
[4] Rushton, P.J. et al. in Plant Cell 14 (2002), p. 749-762 "Synthetic plant promoters containing defined regulatory elements provide novel insights into pathogen- and wound induced signaling"
[5] Ogawa, R. Et al in Biotechniques 42 (2007), p. 628-632: "Construction of strong mammalian promoters by random cis-acting element elongation."
[6]Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994)
[7] del Val C et al in Theor Chem Acc (in press): "PromoterSweep: a tool for identification of transcription factor binding sites.
[8] Stemmer, W.P.C. Et al in Gene 164 (1995), p. 49-53 "Single-step assembly of a gene and entire plasmid from large numbers of oligodeoxyribonucleotides"

### Figure Legends

Fig. 1: Density curves of VDR vs. SREBP
Fig. 2: Spatial distribution of VDR binding sites within 140 natural promoter sequences. 1000 is the transcriptional startsite, 0 is 1000 basepairs upstream of it.
Fig. 3: Frequency of other transcription factors occuring together with VDR. 680 transcription factors wereexamined, of which the displayed 340 show coincidence at least once.

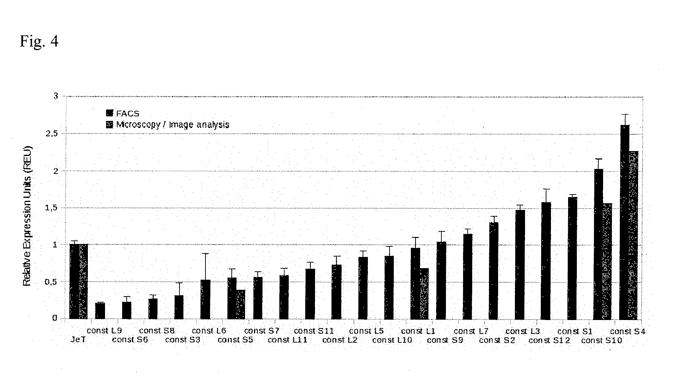

Fig. 4: Activity of 23 clones of constitutive promoters in HeLa cells. Cells were transfected for 16h, medium was replenished and flourescence was measured by flow cytometry 2 hours afterwards. Fluorescence is given in relative value, compared to JeT, a well-described synthetic constitutive promoter.
Fig. 5: Induction characteristics of NFkB-inducible promoters synthesized by RA-PCR. Promoters were induced by 2,5µM TNF-α for 10h in U20S cells, then fluorescence was measured by TECAN. Noise value (transformation with pcDNA5/FRT (Invitrogen) was substracted and fluorescence was plotted in relativevalues to JeT[8] ± TNF-α.
Fig. 6: Induction characteristics of p53-induciblepromoters synthesized by RA-PCR. Promoters were induced by 2,5µM CPT for 12h in MCF-7 cells (or inhibited by Pifithrin-α), then fluorescence was measured by TECAN. Noise value (transformation with pcDNA5/FRT [Invitrogen]) was substracted and fluorescence was plotted in relative values to JeT[8] ± CPT/Pifithrin-α

### Examples:

### Example 1: RA-PCR for synthesis of a promoter responsive to a single stimulus, but of varying strength, protocol

1. Obtain density curves about the distribution of your transcription factor (TF) of interest from our model. Ifthis densitiy curve shows a decisive peak at distance >250Bp from the Transcriptional Start Site (TSS), continue with Example 2 (M-RA-PCR). If a peak is present close to the TSS,or if data is insufficient, continue here.
2. Check our model for transcription factors coinceding with your transcription factor of interest
3. Design two annealing sites, each 15-18 base pairs long. Annealing sites should be void of transcription factor binding sites. Calculate the reverse complement of both sequences. The following sequences were used in this example:

| | Forward (F) | Reverse Complement (RC) |
|---|---|---|
| Annealing Sequence 1 (AS1) | GGGTGACGGGTTCA | AGTGAACCCGTCACCC |
| Annealing Sequence 2 (AS2) | GCGATCGGCAGATCA | TGATCTGCCGATCGC |

4. Design a 5' stop oligos containing a cutsite (SpeI) and AS1_F. The following oligo was used: Stop: 5' CAGTACTAGTGGGTGACGGGTT CA
5. Design a 3' stop oligos containing a cutsite (HindIII) and AS1_RC. The following oligowas used: Stop 3' TGACAAGCTTAGTGAACCCGTCA CCC
6. Design forward and reverse Oligos for each transcription factor of interest. Forward oligos contain AS2_F, the transcription factor binding site and AS1_F. Reverse oligos contain AS2_RC, the TFBS and AS1_RC. Trancription factor binding sites (TFBS) should be designed to represent the matrix describing the factor's binding preferences.
Considering NF-kappaB's frequency matrix, the oligo can be designed in order to represent this matrix, instead of a static NF-kappa B binding site. A sensible representation of this matrix would be GGGRHTTYCC (according to the IUPAC nucleotide code). Most oligonucleotide manufacturers provide the option to synthesize such mixtures of individual oligonucleotides.
For the examples shown below, the following oligos were used:
NFkB forward, version 1:
   GCGATCGGCAGATCAGGGGACTTTGCCGGGTGACGGGTTCA
NFkB forward, version 2:
   GCGATCGGCAGATCAGGGGRWYYCCCGGGTGACGGGTTCA
NfkB reverse: TGATCTGCCGATCGCCCGTTTCAGGGGTGAACCCGTCACCC
p53 , forward:
   GCGATCGGCAGATCAGAACATGTCCAAGCATGCTGGGGTGACGGGTTCA
p53, reverse:
   TGATCTGCCGATCGCGTCGTACGAACCTGTACAAGTGAACCCGTCACCC
7. Design forward and/or reverse oligos for coinceding transcription factors identified in step 2 in the same way as described in step 6.
8. Design forward and/or reverse oligos for general activators. The following oligos were used here:
Spl-responsive (forward):
   GCGATCGGCAGATCAGGGGCGGGGCGGGTGACGGGTTCA
Apl-responsive (forward): GCGATCGGCAGATCATGACTCAGGGTGACGGGTTCA
CREB-responsive (forward):
   GCGATCGGCAGATCABNBVNTGACGTCAGGGTGACGGGTTCA
NFY-responsive (forward):
   GCGATCGGCAGATCANNCCAATNNGGGTGACGGGTTCA
9. Design forward and reverse spacer oligos, which contain 10-15 *N (random nucleotide) instead of a TFBS. The following oligos were used: Spacer_Forward, GCGATCGGCAGATCANNNNNNNNNNGGGTGACGGGTTCA and Spacer_Reverse, TGATCTGCCGATCGCNNNNNNNNNNTGAACCCGTCACCC
10.0rder oligos at 100µM. Pool the oligos. As a general rule, use 0,8µL oft Stop5' and Stop 3'; ∼4µL of the transcription factor (forward), ∼4µL of the transcription factor (reverse), 1-2µL each of the forward and reverse spacer oligo, ∼1µL of coinceding transcription factors and a total of 0, 5µL of general activators. For the examples shown below, the following mixtures of oligos were used:

| | |
|---|---|
| Constitutive | 2µL Sp1-responsive (fwd); 1µL Ap1-responsive (fwd) |
| | 1µL CREB-responsive (fwd); 0.5 NfkB (fwd) version 1; |
| | 0.5 NfkB (fwd) version 1; 4µL Spicer_reverse |
| | 1µL NfkB (rev); 1µL Stop 5; 1µL Stop 3 (0.58) |
| NfkB | 3µL NfkB (fwd) version 1; 3µL NfkB (fwd) version 2 |
| | 4µL NfkB (rev); 3µL Spacer_reverse; 0,2 µL each Sp1, Ap1, |
| | CREB and NFY-responsive; 0.8µL Stop 5 and 0.8µL Stop 3 |
| p53 | 6µL p53-responsive (fwd); 5µL p53-responsive |
| | 1µL random (rev); 0,2 µL each Sp1, Ap1, CREB and NFY-responsive; 0.8µL Stop 5 and Stop 3 |

11. Introduce the oligos thus pooled into a PCR reactions at a final dilutionof 1:200-1:500. Phusion MasterMix 2x (Finnzymes) was used as PCR reagent. Do that PCR reaction twice in order to achieve greater heterogenety.
12.Run the PCR, 7-10 cycles, with the following setup:
○1 cycle Initial dentaturing, 5 minute 95°C
○ 7-10 cycles assembly: 30 seconds 95°C, 45 seconds 58°C, 45 seconds 72°C
○ Terminal hold, 4°C, forever
13. Remove oligonucleotides by performing a PCR purification using PCR purification kit (QIAGEN) or a gel extraction using Gel extraction kit (QIAGEN)
14.Add PCR reagent (Phusion MasterMix 2x) again. Add 5' stop oligo and 3' Stop oligo, 25pmol (1µL of 1:4 diluted stock).
15.Run the PCR, 25 cycles, with the following setup:
○ 1 cycle Initial dentaturing, 5 minute 95°C
○ 25 cycles amplification 30 seconds 95°C, 45 seconds 68°C, 60 seconds 72°C
○ Terminal hold, 4°C, forever
16.Gel purify PCR products to exclude everything <20OBp. Use a 1% agarose gel, 50V for at least 2h to achieve a good resolution
17. Digest with HindIII and SpeI (or whatever cutsites were included in step 4 and 5). Digest a reporter plasmid containing a core promoter and a reporter gene with the same enzymes.
18. In the current example, the plasmid containing GFP as a reporter and a CMV core promoter was used for this task. Make sure to perform a thorough digest; in addition, digest the plasmid with shrimp alkaline phosphatase or calf intestine phosphatase afterwards. Gel purify the plasmid backbone, PCR purify the digested PCR products.
19. Ligate. Perform a thorough ligation to increase transformation efficiency.
Fermentas T4 DNA Ligase for 5h, 19°C or overnight, 16°C was used here.
20. Transform into comptetent E. Coli cells and plate out. Pick no more than 20 colonies per individual PCR reaction. If more putative promoters are desired, set up several PCR reactions
21. Isolate plasmid DNA from the selected colonies. A QIAGEN Miniprep kit for was used forthis tasked.
22. Recommended step: Test-digest miniprep DNA with the same enzymes used in step 17 to make sure you get plasmid with synthesized promoters of varying length. Length of the inserts (that is, synthetic promoters) should be between 100 and 600 basepairs.
If this is not the case, vary stop oligo concentration in step 10, improve gel purification setup in step 16 or alter PCR conditions in step 12 and 15.
23. Perfom a screening to select functioning clones. For example, transfect clones in triplicates into eukaryotic cells on a 96 well plate by using transfection agents such as EFFECTENE or Lipofectamine. Then, induce the conditions of interest in one replicate, shut them off in a second replicate, and leave control medium on the third replicate. When the pathway is fully active, read flourescence (or luminescence, if a luciferase reporter is used) by a plate reader (TECAN) or other automated methods. The following conditions were used for promoter screening:

| | |
|---|---|
| Constitutive | DMEM (GIBCO), 1% Glucosamin, 1% Pen/Strep, 10% FCS 12h |
| NfkB | Control: 2,1 g/l NaHCO3, 1µM CaCl2 10 µM Hepes-Bnffer, 1 % Pen/Strep in Krebs-Henseleit-Buffer (Sigma-Aldrich) [referred to as "minimal medium"], 10h |
| | Induced: Minimal Medium + 2,5µM TNF-α |
| p53 | Control: Minimal medium+ glucose |
| | inhibited: Minimal medium + Pifithrin-α[11] |
| | Induced: Minimal medium + glucose + Camptothecine |

### Example 2

### RA-PCR can be modified to reflect modelled density curves.

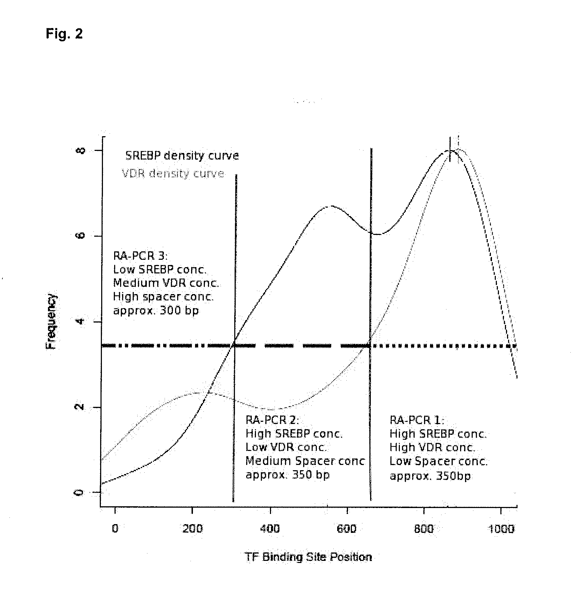

If a promoter regulated by multiple pathways, for example VDR (Vitamin D receptor) and SREBP (Sterol regulated element binding protein), is to be constructed, considering the density curves as obtained from the model (Fig.2) can give clues about its construction. A working VDR/SREBP promoter requires VDR and/or SREBP Res in the close vicinity of the TSS (at approx.850). It might require SREBP REs between300 and 700, and VDR REs between 0 and 300. This distribution can be refelected by setting up 3 RA-PCRs with varying concentrations of VDR-responsive, SREBP responsive and spacer-oligos. If a 3' Stop oligo containing a NheI cutsite, and a 5' Stop oligo containing a SpeI cutsite (or any combination of cutsites yielding compatible ends) is used, an infinite number of RA-PCR products can be assembled and cloned in front of a core promoter (having a SpeI cutsite 5').

### Example 3

### Development of a model describing transcription factor binding

Based on the assumption that transcription factors (TFs) have a spatial preference for binding to the natural promoters' sequence concerning the distance to the transcriptional start site(TSS), we developed HEARTBEAT. In a first step 4395 human promoter sequences 1000 bp upstream from the TSS obtained from theUCSC genome browser were analysed by the program "Promotersweep" [7].Promotersweep is able to assign transcription factor binding sites (TFBS) to a given sequence by retrieving and combining information from three homology databases (EnsEMBL Compara, NCBI HomoloGene,DoOP database), five promoter databases(EPD, DBTSS), six sequence motive identification tools (e.g. Meme, Gibbs MotifSampler) and two matrix profile databases (Jaspar Core Library, Transfac ProfessionalLibrary). Each TFBS motive is further classified into weak, conserved and reliablea ccording to the quality of the assignment.

The final result of Promotersweep can be divided into general spatial information about the TFBS and the consensus sequence on the one hand and further detailed facts about the associated gene on the other. This partially redundant data is stored into two MySQL-tables, which allow fast and sophisticated queries. In the "Main_Info"-table a TFBS is characterised by the TF name, motive start bp, motive end bp, Refseq ID and the associated TF matrix from Transfac.

In the table "Gene_Info" one can gain information about the Ensemble ID; a short gene description; different possible TSS fromDoOP, DBTSS, EPD and MpromDB. With a statistical analysis based on the programming language R we were able to create histograms illustrating the distribution for each TFBS. To decrease the influence of outliers we calculated for every histogram a probability density function which is further used in the RA-PCR method.

### Example 4

### A model describing transcription factor binding preferences and coincidence

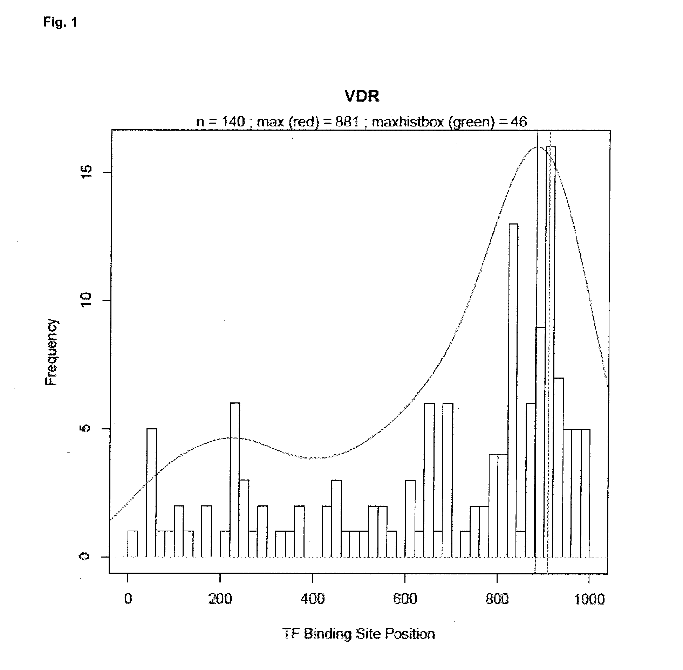

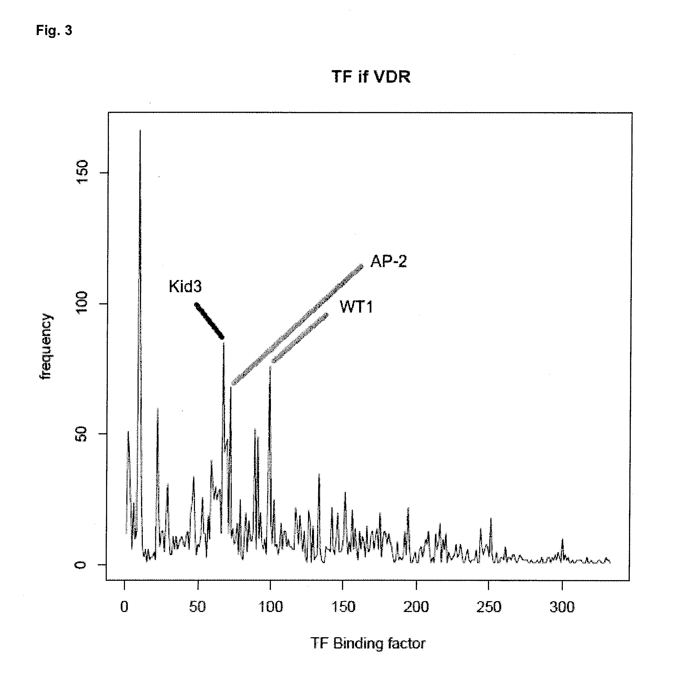

In Fig. 1 the spatial distribution of VDR binding sites within 140 natural promoter sequences is shown as an example. The size of each bin equals the number of VDR-TFBS within a range of 20 bps. The solid line represents the probability density function (pdf). Here, the maximum of the pdf is located 54 bps upstream to the TSS indicated by the vertical line. Natural promoter sequences usually exhibit multiple TFBS which implies dependencies between different TFs according to their binding behaviour to the DNA. Fig. 3 shows the frequency distribution of coincidental appearing TFBSs if VDR is present. The highest peak represents VDR itself. The next three highest peaks are Kid3 (inhibitory), WT1 and AP-2 (stimulatory). In total, together with VDR, there are over 300 different TFBS coincidentally present. Both plots represent data deduced from the Heartbeat-database which enable a welldefined synthesis of promoter sequences.

### Example 5

### Synthesis of a library of constitutive promoters of varying strength

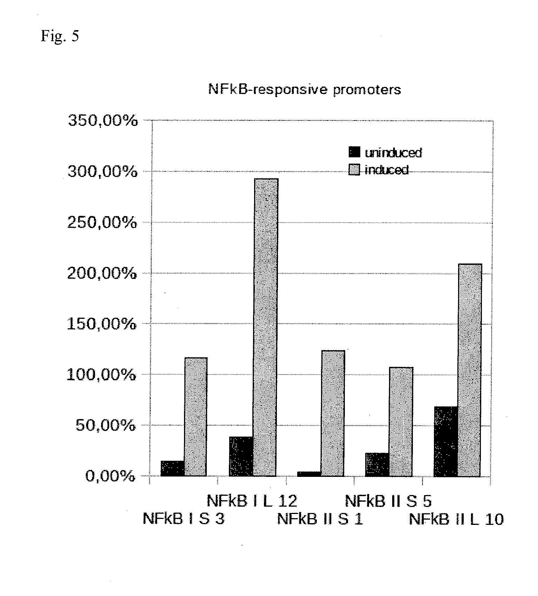

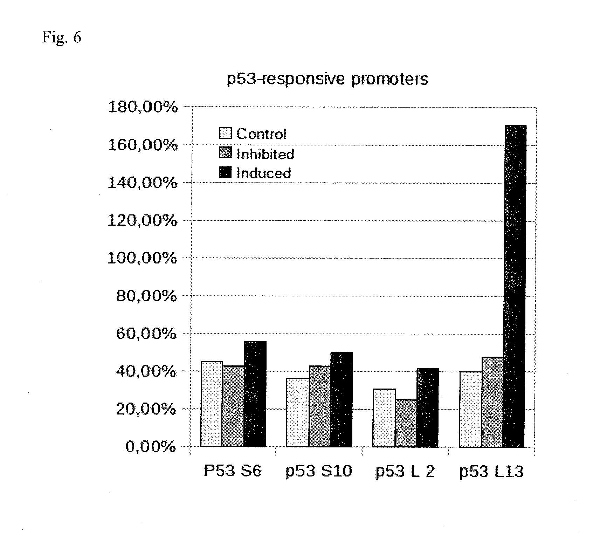

By performing RA-PCR with a mixture of general activators, a library of constitutively active promoters of different strength (Fig 5) was synthesized. As sequence analysis (Fig 6) shows, RA-PCR creates randomized repeats of the oligos inserted into the reaction.

### Example 6

### Synthesis of a library of stimuli-responsive promoters of varying strength and inducibility

By performing RA-PCR with mixtures of Oligos which were mostly responsive to NFkB (nuclearfactor kappa B) and p53 (compare material and methods), promoters with different induction and repression characteristics were obtained, as characterized by a plate reader measurement in relative values to JeT[8] (Fig. 5, 6). Table 1 shows sequences of NfkB inducible promoters.

**Table 1: Examples of promoters:**

| CONSTITUTIVE PROMOTERS | |
|---|---|
| Const L 1 | ACTAgtGGGTGACGGGTTCACCCCTGAAACGGGCGATCGGCAGATCAGGGGACTTTGCCGGG TGACGGGTTCAATGACCGATCAGCGATCGGCAGATCAGGGGACTTTGCCGGGTGACGGGTTC ACTCAACAGACAGCGATCGGCAGATCATCCAGTGACGTCAGGGTGACGGGTTCACCGCATAC AGCGCGATCGGCAATCACTGAGTGACGTCAGGGTGACGGGTTCACTAAGCTT |
| Const L 4 | ACTAGTGGGTGACGGGTTCAGCTGTGCGTGGGCGATCGGCAGATCAGGGGACTTTGCCGGGT GACGGGTTCACAGCTTAGTCAGCGATCGGCAGATCAGGGGATTTCCCGGGTGACGGGTTCAC GTTACACGGCGCGATCGGCAGATCATGACTCAGGGTGACGGGTTCAGTTGGGACCATGCGAT CGGCAGATCATGACTCAGGGTGACGGGTTCACTAAGCTT |
| const L 5 | ACTAGTGGGTGACGGGTTCAGGTCCACACACGCGATCGGCAGATCACTTATTGACGTCAGGG TGACGGGTTCACTAAGCTT |
| const S 5 | ACTAGTGGGTGACGGGTTCATATTGTTAAAGGCGATCGGCAGATCAGGGGGTCCCCCGGgtG ACGGGTTCACTTAGTCAGGTGCGATCGGCAGATCATGACTCAGGGTGACGGGTTCACTAAGC TT |
| const S 10 | ACTAGTGGGTGACGGGTTCACTAGCGCAAACGCGATCGGCAGATCAGGGGATCTCCCGGGTG ACGGGTTCAGGGCAGTCTGAGCGATCGGCAGATCATTGACTGACGTCAGGgtGACGGGTTCA ACATGCGTAGCGCGATCGGCAGATCAGGGGCGGGGCGGGTGACGGGTTCACACATGCTTTGG CGATCGGCAGATCAGATGGTGACGTCAGGGTGACGGGTTCAATTAACTCCCAGCGATCGGCA GATCAGGGGACTTTGCCGGGTGACGGGTTCATAGACTAACGGGCGATCGGCAGATCATGACT CAGGGTGACGGGTTCACTAAGCTT |
| const S 4 | ACTAgtGGGTGACGGGTTCATTTGACAAGATGCGATCGGCAGATCATTTGTTGACGTCAGGG TGACGGGTTCACATACACACAGGCGATCGGCAGATCAGGGGAACCCCCGGGTGACGGGTTCA CAACCAGGgATGCGATCGGCAGATCAGTTATTGACGTCAGGGTGACGGGTTCACTAAGCTT |

| NFkB RESPONSIVE PROMOTER | |
|---|---|
| clone NIIL10 (best NFkB resposnive promoter) | ACTAGTGGGTGACGGGTTCAGTGACCACGCAGCGATCGGCAGATCAGGGGGTTCCCCGGGTG ACGGGTTCATGTCAGCAGGGGCGATCGGCAGATCATACGGTGACGTCAGGGTGACTGGTTCA ATCACGTGAATGCGATCGGCAGATCAGGGGGATTCCCGGGTGACGGGTTCACGACAAGCGCG GCGATCGGCAGATCAGGGGGTTCCCCGGGTGACGGGTTCACCCCTGAAACGGGCGATCGGCA GATCAAGCCAATGGGGGTGACGGGTTCACTAAGCTT |

| other promoters: | |
|---|---|
| 3 | ACTAGTGGTGACGGGTTCACCCCTGAAACGGGCGATCGGCAGATCAGGTTCACACTGGGTGA CGGGTTCACTAAGCTT |
| 11 | ACTAGTGGGTGACGGGTTCATCAAAACGGACGCGATCGGCAGATCAGGGGGACCCCCGgtGA CGGGTTCACTAAGCTT |
| 12 | ACTAGTGGGTGACGGGTTCAATAACGCAAAGGCGATCGGCAGATCATGGCTTGACGTCAGGG TGACGGGTTCACTCAGGCAGAGCGATCGGCAGATCAGGGGACTTTGCCGGGTGACGGGTTCA CTAAGCTT |
| 16 | ACTAGTGGGTGACGGGTTCACCCCTGAAACGGGCGATCGGCAGATCAGGGGACTTTGCCGGG TGACGGGTTCACTAAGCTT |
| 32 | ACTAGTGGGTGACGGGTTCAGTGACCACGCAGCGATCGGCAGATCAGGGGGTTCCCCGGGTG ACGGGTTCATGTCAGCAGGGGCGATCGGCAGATCATACGGTGACGTCAGGGTGACTGGTTCA ATCACGTGAATGCGATCGGCAGATCAGGGGGATTCCCGGGTGACGGGTTCACGACAAGCGCG GCGATCGGCAGATCAGGGGGTTCCCCGGGTGACGGGTTCACCCCTGAAACGGGCGATCGGCA GATCAAGCCAATGGGGGTGACGGGTTCACTAAGCTT |
| 14 | ACTAgtGGGTGACGGGTTCACCCCTGAAACGGGCGATCGGCAGATCAGGGGAATCCCCGGgt GACGGGtTCACCCCTGAAACGGGCGATCGGCAGATCAGGGGGACTCCCGGgtGACGGGTTCA TCGCCCCAGCGGCGATCGGCAGATCAGCGAGAGTTTGGGTGACGGGTTCAACAGCAGTAGCG CGATCGGCAGATCAGGGGGTTCCCCGGGTGACGGGTTCAATTCCCTAGTAGCGATCGGCAGA TCATTTATTGACGTCAGGGTGACGGGTTCACCCCTGAAACGGGCGATCGGCAGATCAGGGGA |
| | CTTTGCCGGGTGACGGGTTCACTAAGCTT |
| 17 | AcTagtGGGTGACGGGtTCACCCCTGAAACGGGCGATCGGCAGATCAGGGGACTTTGCCGGg tGACGGGtTCACCCCTGAAACGGGCGATCGGCAGATCAGGTATTGACGTCAGGGTGACGGGT TCACATCATTCTCAGCGATCGGCAGATCAGTTCTTGACGTCAGGGTGACGGGTTCACTAAGC TT |
| 23 | AcTagtGGGTGACGGGTTCAGATTTTACACCGCGATCGGCAGATCATGACTCAGGGTGACGG GTTCAACTGACAAGACGCGATCGGCAGATCAATGAGGTTGGGGGTGACGGGtTCACCCCTGA AACGGGCGATCGGCAGATCAGTGAGTGACGTCAGGGTGACGGGTTCACCCCTGAAACGGGCG ATCGGCAGATTAGTTGCTGACGTCAGGGTGACGGGTTCACTAAGCTT |

## Claims

1. A method for the manufacture of a polynucleotide comprising a synthetic promoter specifically controlled by at least one selected transcription factor, comprising
a) determining the distance between the transcriptional start point and the binding site for said at least one transcription factor in a given number of promoters comprising at least one binding site for said at least one transcription factor,
b) selecting a distance range comprising the distances determined in a) to occur most frequently,
c) determining the accompanying transcription factors in a given number of promoters,
d) selecting a suitable number of accompanying transcription factors determined in c) to occur most frequently,
e) synthesizing the synthetic promoter by randomly assembling
i) at least one binding site for each of the at least one transcription factor,
ii) at least one binding site for each of the selected accompanying transcription factor(s), and
iii) a suitable number of stuffer fragments
and by arranging said randomly assembled binding sites in the distance range from the transcriptional start point selected in b).

2. The method of claim 1, wherein said distance between the transcriptional start point and the binding site is determined by a genome-wide analysis of promoters comprising at least one binding site for said at least one transcription factor.

3. The method of claim 1 or 2, wherein the accompanying transcription factors in a given number of naturally occurring promoters are determined by a genome-wide coincidence analysis of said at least one transcription factor.

4. The method of any one of claims 1 to 3, wherein the synthetic promoter is synthesized by PCR.

5. The method of claim 4, wherein the PCR is random assembly PCR.

6. A polynucleotide comprising the synthetic promoter manufactured by the method of any one of claims 1 to 5.

7. A vector comprising the polynucleotide of claim 6.

8. A host cell comprising the polynucleotide of claim 6, or the vector of claim 7.

9. A device for the manufacture of oligonucleotides suitable for manufacturing a synthetic promoter, comprising
a) a database comprising data from a coincidence analysis of transcription factors and
b) a synthesis unit synthesizing oligonucleotides, wherein the nucleotide sequence of said oligonucleotides is determined using data comprised in the database of a).

10. A device for the manufacture of a synthetic promoter, comprising
a) a database comprising data from a localisation analysis of transcription factors,
b) a database comprising data from coincidence analysis of transcription factors, and
c) a synthesis unit synthesizing a polynucleotide comprising a synthetic promoter,
wherein the nucleotide sequence of said polynucleotide is determined using data comprised in the databases of a) and b).

11. The device of claim 11, wherein the synthesis unit comprises the device of claim 10.
